# EUROPÄISCHE PATENTANMELDUNG

(11) **EP 0 706 228 A1**
(43) Veröffentlichungstag der Anmeldung: **10.04.1996**
(21) Anmeldenummer: 95250245.8
(22) Anmeldetag: 06.10.1995
(51) Int. Cl.: H01L 51/40, H01L 31/04

(54) **Verfahren zur Herstellung eines Bio-Sensors für elektromagnetische Strahlung**

(30) Priorität: 08.10.1994 DE 4437023
(71) Anmelder: INSTITUT FÜR BIOPROZESS- UND ANALYSENMESSTECHNIK e.V., D-37308 Heiligenstadt (DE)
(72) Erfinder: Gruber, Ronald, Dipl.-Biochem., D-37308 Geisleden (DE); Beckmann, Dieter, Dipl.-Phys., D-37308 Heiligenstadt (DE); Müller, Adrian, Dipl.-Ing., D-37308 Heiligenstadt (DE); Klingebiel, Ursel, D-37308 Heiligenstadt (DE)
(74) Vertreter: Ninnemann, Detlef, Dipl.-Ing.

(57) **Zusammenfassung**

Die Erfindung betrifft ein Verfahren zur Herstellung eines Bio-Sensors für elektromagnetische Strahlung. Erfindungsgemäß wird eine Suspension eines strahlungsempfindlichen biologichen Materials auf eine die Orientierung des biologischen Materials realisierende Membran aufgebracht. Anschließend wird dieser Anordnung das Lösungsmittel entzogen und diese Anordnung wird dann zwischen zwei flächige Elektroden gebracht, von denen mindestens eine für elektromagnetische Strahlung durchlässig ist. Als Membran wird vorzugsweise eine Kernspurmembran verwendet. Bei Anwendung dieses Verfahrens liegt im Gegensatz zu bekannten Präparationen das strahlungsempfindliche biologische Material auf der Oberfläche oder in Poren einer Membran vor. Damit ist keine chemische Präparation und keine Ausrichtung in einem elektrischen oder magnetischen Feld erforderlich.

## Beschreibung

Die Erfindung betrifft ein Verfahren zur Herstellung eines Bio-Sensors für elektromagnetische Strahlung nach dem Oberbegriff des Anspruchs 1.

Bio-Sensoren für elektromagnetische Strahlung sind bekannt. Sie enthalten in der Regel photoempfindliches biologisches Material, das orientiert auf einem Substrat aufgebracht ist.

Neben einer bioelektrischen Anordnung, bei der ein photoempfindlciehs Protein in einer Membran orientiert eingeschlossen und zur Stabilisierung der Moleküle mit einem Polymer-Film bedeckt ist, bzw. bei der die orientierten Membranmoleküle durch Zusatz bestimmter Substanzen und mit Hilfe bestimmter Verfahren miteinander vernetzt werden, ist es bekannt, als strahlungsempfindliches biologisches Material rhodopsinhaltige Mikroorganismen oder Zellen zu verwenden (DE 42 24 602 C1). Die letztgenannte Anordnung weist gegenüber den vorgenannten den Vorteil auf, daß der gleiche strahlungstechnische Effekt ohne zusätzliche Maßnahmen zur Orientierung der Moleküle erreicht wird.

Der gleiche Vorteil wird dadurch erzielt, daß in einem lyotropen Flüssigkristall eingebettetes strahlungsempfindliches biologisches Material verwendet wird.

Der Erfindung liegt die Aufgabe zugrunde, Orientierung und Stabilisierung von strahlungsempfindlichem biologischem Material weiter zu vereinfachen.

Erfindungsgemäß wird das entsprechend den kennzeichnenden Merkmalen des Anspruchs 1 erreicht.

Bei einem Verfahren zur Herstellung eines Bio-Sensors für elektromagnetische Strahlung wird erfindungsgemäß eine Suspension eines strahlungsempfindlichen biologichen Materials auf eine die Orientierung des biologischen Materials realisierende Membran aufgebracht. Anschließend wird dieser Anordnung das Lösungsmittel entzogen und sie wird zwischen zwei flächige Elektroden gebracht, von denen mindestens eine für elektromagnetische Strahlung durchlässig ist.

Bei Anwendung dieses Verfahrens liegt im Gegensatz zu bekannten Präparationen das strahlungsempfindliche biologische Material auf der Oberfläche oder in Poren einer Membran vor. Damit ist keine chemische Präparation und keine Ausrichtung in einem elektrischen oder magnetischen Feld erforderlich.

In einer Ausführungsform wird eine Suspension des strahlungsempfindlichen biologischen Materials auf ein poröses Trägermaterial aufgebracht. Anschließend wird das Lösungsmittel durch das poröse Material abgesaugt.

In einer weiteren bevorzugten Ausführungsform wird als Membran, die eine Orientierung des biologischen Materials realisiert, eine Kernspurmembran verwendet.

Zusätzlich kann das strahlungsempfindliche biologische Material nach Entzug des Lösungsmittels in einen Flüssigkristall eingebracht werden. Dieses Präparat wird anschließend als dünne Schicht auf die Membran aufgebracht. Damit ist es möglich, eine größere Menge des strahlungsempfindlichen biologischen Materials einzubringen, wodurch eine Verstärkung des Meßeffektes erreicht wird. Im Gegensatz dazu würde das Aufbringen mehrerer Schichten auf ein Trägermaterial ohne Einschluß in einen Flüssigkristall den Meßeffekt verringern, da weitere Schichten durch fehlenden Kontakt zum Trägermaterial keine Vorzugsrichtung mehr aufweisen. Der Vorteil dieser Anordnung besteht außerdem darin, daß das strahlungsempfindliche biologische Material vor äußerem chemischen oder biologischen Einfluß geschützt ist..

Die Komponenten des strahlungsempfindlichen biologischen Materials und des Flüssigkristalls werden mit der Membran in Kontakt gebracht. Nach Ausbildung des Flüssigkristalls auf der Oberfläche und in den Poren der Membran wird die so entstandene Anordnung zwischen die Elektroden gebracht.

Als strahlungsempfindliches biologisches Material wird vorzugsweise Bakteriorhodopsin verwendet.

Die Erfindung soll in einem Ausführungsbeispiel erläutert werden.

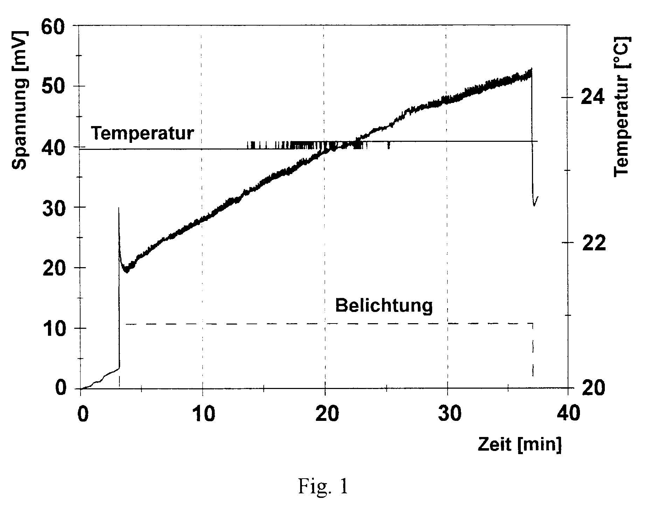

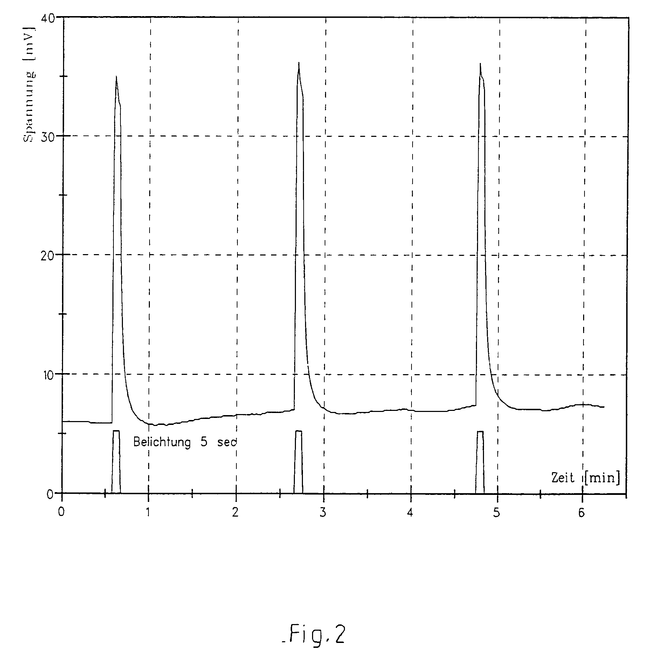

Die Figuren 1 und 2 zeigen die Meßergebnisse mit Sensoren, die nach dem nachfolgend beschriebenen erfindungsgemäßen Verfahren hergestellt wurden.

Eine Bakteriorhodopsinlösung wird in einer Menge von 1 mg/cm² auf eine Kernspurmembran aufgebracht. Die Kernspurmembran weist eine Dicke von 10 µm, eine Porengröße von 200 nm sowie 5 Mio Poren/cm² auf.

Durch das Ansaugen mit einer Vakuumpumpe von der Gegenseite der Kernspurmembran wird das Bakteriorhodopsin auf der Oberfläche der Membran und in den Poren abgelagert. Das Lösungsmittel wird senkrecht zur Membran durchgesaugt und abgeführt. Es entsteht eine zusammenhängende Bakteriorhodopsinschicht auf der Membranoberfläche, die in die Poren übergeht.

Zur Kontaktierung wird die präparierte Membran zwischen zwei Glasplatten gebracht, die mit leitfähigem Indium-Zinn-Oxid transparent beschichtet sind.

Zum Nachweis des bioelektrischen Effekts wurde an die leitfähigen Schichten ein hochohmiger Meßverstärker (Rₑ=10₁₂ Ohm) angeschlossen. Zur Belichtung wurde eine 75 W-Xenon-Bogenlampe verwendet. Die Wärmestrahlung wurde mit einem Reflexionsfilter ausgeblendet. Die Übertragung zur Probe erfolgte mit einem Flüssigkristall-Lichtleitkabel (Durchmesser 3 mm, Länge 1,7 m). Das Lichtleitkabel wurde so auf die Membran gerichtet, daß der Bakteriorhodopsinfilm auf der Membran gleichmäßig ausgeleuchtet wurde.

Mit dieser Meßanordnung wurde das in der Fig. 1 dargestellt te impulsförmige Signal erzeugt. Hierbei erfolgte die Belichtung ebenfalls impulsförmig.

Das in Fig. 2 dargestellte Signal erhält man bei Dauerbeleuchtung.

## Patentansprüche

1. Verfahren zur Herstellung eines Bio-Sensors für elektromagnetische Strahlung,
**dadurch gekennzeichnet,**
daß eine Suspension eines strahlungsempfindlichen biologichen Materials auf eine die Orientierung des biologischen Materials realisierende Membran aufgebracht wird, daß anschließend dieser Anordnung das Lösungsmittel entzogen und daß diese Anordnung zwischen zwei flächige Elektroden gebracht wird, von denen mindestens eine für elektromagnetische Strahlung durchlässig ist.

2. Verfahren nach Anspruch 1, **dadurch gekenzeichnet,** daß die Suspension des strahlungsempfindlichen biologischen Materials auf ein poröses Trägermaterial aufgebracht wird und daß anschließend das Lösungsmittel durch das poröse Material abgesaugt wird.

3. Verfahren nach Anspruch 1 oder 2, **dadurch gekennzeichnet,** daß als Membran eine Kernspurmembran verwendet wird.

4. Verfahren nach mindestens einem der vorhergehenden Ansprüche, **dadurch gekennzeichnet,** daß das strahlungsempfindliche biologische Material nach Entzug des Lösungsmittels in einen Flüssigkristall eingebracht und daß dieses Präparat anschließend als dünne Schicht auf die Membran aufgebracht wird.

5. Verfahren nach mindestens einem der vorhergehenden Ansprüche, **dadurch gekennzeichnet,** daß die Komponenten des strahlungsempfindlichen biologischen Materials und des Flüssigkristalls mit der Membran in Kontakt gebracht werden und daß nach Ausbildung des Flüssigkristalls auf der Oberfläche und in den Poren der Membran die so entstandene Anordnung zwischen die Elektroden gebracht wird.

6. Verfahren nach mindestens einem der vorhergehenden Ansprüche, **dadurch gekennzeichnet,** daß als strahlungsempfindliches biologisches Material Bakteriorhodopsin verwendet wird.

7. Verfahren nach mindestens einem der vorhergehenden Ansprüche, **dadurch gekennzeichnet,** daß eine Bakteriorhodopsinlösung in einer Menge von 1 mg/cm² auf eine Kernspurmembran aufgebracht wird, die eine Dicke von 10 µm, eine Porengröße von 200 nm sowie 5 Mio Poren/cm² aufweist.

8. Verfahren nach mindesten einem der vorhergehenden Ansprüche, **dadurch gekennzeichnet,** daß die präparierte Membran zur Kontaktierung zwischen zwei Glasplatten gebracht wird, die mit leitfähigem Indium-Zinn-Oxid transparent beschichtet sind.
